# FASCICULE DE BREVET EUROPEEN

(11) **EP 2 581 108 B1**
(45) Date de publication et mention de la délivrance du brevet: **23.10.2013**
(21) Numéro de dépôt: 12169932.6
(22) Date de dépôt: 30.05.2012
(51) Int. Cl.: A61N 1/08, A61N 1/05, A61N 1/372, B82Y 10/00, H03K 17/24, A61N 1/36, A61N 1/368, G11C 17/00, G11C 13/02

(54) **Module de commutation contrôlée de sonde multiélectrode pour un dispositif médical implantable actif**
Über eine multiplexierte Sonde gesteuertes Schaltmodul für ein implantierbares aktives medizinisches Gerät
Module for controlled switching of a multielectrode probe for an active implantable medical device

(30) Priorité: 13.10.2011 FR 1159288
(43) Date de publication de la demande: 17.04.2013
(73) Titulaire: Sorin CRM SAS, 92140 Clamart Cedex (FR)
(72) Inventeur: Amara, Karima, 92330 Sceaux (FR); Seoudi, Islam, 92290- Châtenay-Malabry (FR)
(74) Mandataire: Dupuis-Latour, Dominique

(56) Documents cités:
- EP-A1- 1 938 861
- EP-A1- 2 082 684
- US-A1- 2009 118 790
- US-A1- 2009 198 313
- US-A1- 2010 312 298
- US-A1- 2011 029 042

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

Ces dispositifs comportent un boîtier généralement désigné "générateur", raccordé électriquement et mécaniquement à une ou plusieurs "sondes" munie(s) d'électrodes destinées à venir en contact avec les tissus à stimuler et/ou sur lesquels on souhaite recueillir un signal électrique : myocarde, nerf, muscle ... Dans le cas d'un dispositif de diagnostic et de thérapie cardiaque, ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

Un premier aspect du développement de ces appareils est la multiplication du nombre d'électrodes, notamment pour les appareils dits "multisite" qui permettent de choisir les sites de stimulation/détection optimisant le fonctionnement de l'appareil.

Ainsi, dans le cas particulier des dispositifs de resynchronisation ventriculaire (dispositifs dits CRT, *Cardiac Resynchronization Therapy*), on implante au patient un appareil muni d'électrodes permettant de stimuler l'un et l'autre ventricule. La stimulation du ventricule droit (et de l'oreillette droite) est opérée par une sonde endocavitaire classique, mais pour le ventricule gauche l'accès est plus complexe ; la stimulation est alors généralement opérée au moyen d'une sonde introduite dans le sinus coronaire du ventricule droit puis poussée dans une veine coronaire sur l'épicarde, de façon que l'extrémité de la sonde vienne se placer face au ventricule gauche. Cette procédure est assez délicate, car le diamètre des vaisseaux coronaires se réduit avec l'avancement de la sonde, de sorte qu'il n'est pas toujours facile de trouver la position optimale lors de l'implantation. De plus, la proximité du nerf phrénique peut parfois conduire à des stimulations inappropriées.

C'est pour pallier ces difficultés que les spécialistes ont été conduits à développer des sondes dites "multiélectrode", pourvues par exemple de dix électrodes et dont il est possible de choisir après implantation l'électrode de stimulation la plus efficace. Une telle sonde est par exemple décrite dans le EP 1 938 861 A1 (ELA Medical).

Pour gérer cette multiplicité d'électrodes, on a développé des systèmes de multiplexage permettant d'interfacer les diverses électrodes (ainsi que les éventuels capteurs portés par la sonde) avec les deux conducteurs parcourant la sonde et reliés aux bornes du générateur.

Le EP 2 082 684 A1 (ELA Medical) décrit ainsi un générateur relié à une sonde multiélectrode par deux conducteurs associés à un circuit multiplexeur/démultiplexeur. Ces deux conducteurs d'une part assurent le recueil des signaux de dépolarisation et l'envoi des impulsions de stimulation, et d'autre part délivrent au multiplexeur/démultiplexeur des signaux logiques permettant de contrôler des commutateurs de sélection d'une ou plusieurs électrodes de la sonde. Ces signaux assurent également la fourniture au circuit de multiplexage/démultiplexage et aux commutateurs de l'énergie nécessaire à leur fonctionnement. Le EP 1 938 861 A1 précité décrit un tel circuit de multiplexage et de commutation contrôlée, ainsi qu'un protocole d'échange de signaux entre le générateur et les différents multiplexeurs de la sonde pour assurer la commutation voulue par envoi de trains d'impulsions spécifiques sur la ligne bifilaire.

Le US 2011/0029042 A1 décrit un autre dispositif comportant un module de commutation contrôlée du même type, avec un interrupteur commandé associé à un composant de mémoire qui stocke un identifiant unique utilisé pour l'adressage par le multiplexeur/démultiplexeur.

Un premier inconvénient de ces dispositifs connus est la nécessité de fournir une alimentation permanente aux circuits qui permettent le multiplexage des actuateurs définissant la configuration des électrodes. Ceci induit une augmentation de la consommation globale du système implant/sonde, préjudiciable à la durée de vie du dispositif implanté.

Plus précisément, pour une sonde multiélectrode donnée telle que celle décrite dans les deux documents précités, les électrodes sont sélectionnées par le générateur via la liaison bifilaire portée par le corps de sonde, dont les deux pôles sont généralement désignés "distal" et "proximal". On notera que dans certains cas, la liaison peut comporter un ou plusieurs conducteurs supplémentaires, la liaison pouvant par exemple être une liaison trifilaire intégrant un conducteur supplémentaire pour la transmission directe au générateur de signaux produits par exemple par un capteur d'accélération endocardiaque situé en bout de sonde.

Un module de circuit, généralement réalisé sous forme d'un circuit intégré spécifique ASIC, est intégré au niveau de chaque électrode. Ce module, qui doit être alimenté via la liaison bifilaire, reçoit les données de configuration permettant de définir si l'électrode correspondante doit être connectée ou non et, dans l'affirmative, à quel pôle, distal ou proximal. Les données de configuration sont interprétées par le module, qui réalise (ou non) la liaison de l'un ou l'autre des conducteurs distal ou proximal à l'électrode choisie, au moyen d'interrupteurs commandés.

Ces interrupteurs commandés sont des interrupteurs volatils, le plus souvent des transistors MOS ou des microsystèmes électromécaniques MEMS.

Or, une fois que les divers modules de la sonde ont été configurés pour activer un ou plusieurs sites de stimulation correspondant aux électrodes respectives, la configuration d'électrodes doit être maintenue en permanence pour permettre le recueil du signal cardiaque et l'application si besoin d'impulsions de stimulation.

Un *premier inconvénient* est ainsi la nécessité de fournir une énergie suffisante de manière permanente ou périodique (c'est-à-dire pendant toute la période où l'implant est actif) pour alimenter les différents modules ASIC de la sonde.

Un *deuxième inconvénient* tient au fait que, pour une sonde donnée, il est nécessaire d'utiliser un implant spécifique, dédié à cette application. En effet, le générateur associé à la sonde doit être capable de fournir les signaux de multiplexage appropriés ainsi que l'énergie d'alimentation permettant de rendre la sonde fonctionnelle.

Un *troisième inconvénient* tient au fait que dans certains cas particuliers la sonde n'est plus alimentée par le générateur, ce qui entraîne la perte de la configuration de stimulation, qui doit être reprogrammée. Tel est en particulier le cas en fin de vie de l'implant (épuisement de la pile), lors du remplacement par un autre implant neuf : pendant l'intervention, la sonde n'est plus alimentée et une fois l'implant neuf mis en place il est nécessaire de reconfigurer la sonde dans son état antérieur - ce qui au surplus suppose que cet état antérieur ait été sauvegardé pendant que l'ancien générateur était encore fonctionnel.

Ainsi, un *premier but* de l'invention est de réduire la consommation d'énergie d'un système de sonde multiélectrode, consommation liée à l'obligation d'alimenter en énergie les modules ASIC afin de maintenir les électrodes de la sonde dans la configuration optimale choisie.

Un *deuxième but* de l'invention est de s'affranchir de la reprogrammation de la configuration de stimulation en cas de changement de générateur, en maintenant cette configuration même en l'absence d'alimentation de la sonde.

Un *troisième but* de l'invention est d'assurer une compatibilité avec un générateur quelconque et une sonde de type multiélectrode. Il s'agit, vue du générateur, de rendre la sonde équivalente à une sonde bipolaire standard, donc compatible avec un générateur de modèle ou de marque différent, dès lors que celui-ci est conforme aux standards de connexion IS-1 (pour une simple configuration bipolaire). L'adaptation à la configuration multiélectrode sera alors réalisée par la sonde elle-même - et non par le générateur, qui peut donc être dépourvu de fonctions de multiplexage dans la mesure où la sonde conservera la configuration d'électrodes définie initialement.

En d'autres termes, l'invention a pour objet de proposer une solution permettant non seulement de réduire la consommation d'énergie mais également de rendre une sonde multiélectrode compatible avec tout type de générateur disponible sur le marché, en garantissant une connexion bipolaire ou unipolaire identique à la dernière configuration choisie avant le remplacement du générateur ayant permis la programmation initiale de la configuration d'électrodes de la sonde.

Plus précisément, l'invention a trait à un module de commutation contrôlée d'électrode du type divulgué notamment par le US 2011/0029042 A1 précité, pour un dispositif médical implantable actif comprenant un générateur et une sonde reliée à ce générateur et pourvue d'une pluralité d'électrodes sélectivement commutables de détection/stimulation.

Ce module comprend : au moins une borne proximale et une borne distale, aptes à être couplées à des conducteurs respectifs d'une ligne bifilaire de la sonde, reliée au générateur ; une borne d'électrode, reliée à l'une des électrodes de détection/stimulation de la sonde ; un circuit de commutation sélective de la borne d'électrode à l'une ou à l'autre des bornes proximale et distale, comprenant au moins un interrupteur commandé couplant la borne d'électrode respectivement aux bornes proximale et distale, et associé à au moins un composant de mémoire non volatile programmable ; et un circuit de décodage pour leur activation une alimentation électrique ; et un circuit de décodage de signaux délivrés par le générateur et transmis par la ligne bifilaire pour commander le circuit de commutation selon une configuration particulière correspondante.

De façon caractéristique de l'invention, il est prévu deux interrupteurs commandés couplant la borne d'électrode respectivement aux bornes proximale et distale, ces interrupteurs commandés étant des interrupteurs volatils nécessitant pour leur activation une alimentation électrique. Le composant de mémoire non volatile programmable possède en l'absence d'alimentation électrique deux états stables, chacun de ces états stables contrôlant un état correspondant ouvert ou fermé de l'interrupteur commandé. Le circuit de décodage comporte des moyens pour programmer préalablement l'état du composant de mémoire non volatile par des signaux correspondants appliqués par le générateur sur la ligne bifilaire. Le circuit de commutation comprend en outre un circuit générateur de maximum-minimum, couplé en entrée entre les deux bornes proximale et distale, et couplé en sortie aux interrupteurs commandés pour commander sélectivement ceux-ci via le composant de mémoire non volatile correspondant.

Dans une forme de réalisation avantageuse, les deux interrupteurs commandés sont des interrupteurs complémentaires commandés par application d'une tension à une borne de pilotage commune, de sorte que lorsque l'un des interrupteurs est commandé ouvert l'autre soit commandé fermé, et *vice versa.*

Dans ce cas, le module peut comprendre deux composants de mémoire non volatile montés en série entre une sortie de minimum et une sortie de maximum du générateur de maximum-minimum. L'un des composants de mémoire non volatile est programmé dans l'un de ses états stables et l'autre dans l'état stable opposé. Le point milieu du montage série est relié à la borne de pilotage commune des interrupteurs complémentaires.

Le composant de mémoire non volatile programmable peut notamment être une cellule à nanotube suspendu, ou bien une cellule à jonction tunnel magnétique.

L'invention porte également sur une sonde en tant que telle, incorporant un module de commutation contrôlée tel que ci-dessus.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre, de façon schématique, un exemple de dispositif médical comportant un générateur et trois sondes implantées dans trois cavités respectives du myocarde.
La Figure 2 illustre les différents modules couplés à la liaison bifilaire entre le générateur et l'extrémité distale de l'une des sondes de l'ensemble de la Figure 1, avec les électrodes commutables respectives.
La Figure 3 illustre de façon plus détaillée les divers composants et blocs fonctionnels des modules de commutation représentés schématiquement sur la Figure 2.

On va maintenant décrire un exemple, non limitatif, de réalisation de l'invention.

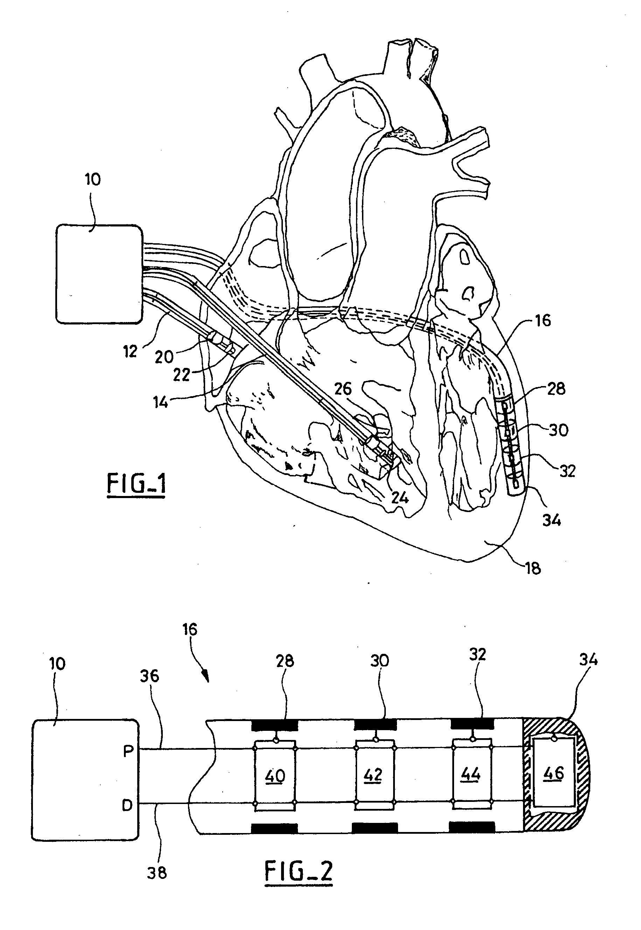

Sur la Figure 1, la référence 10 désigne le boîtier de générateur d'un dispositif de type défibrillateur, stimulateur ou resynchroniseur. On notera que cette application n'est aucunement limitative, l'invention pouvant s'appliquer à d'autres implants tels que dispositifs de détection/stimulation de nerf, de muscle, etc.

Le générateur 10 est associé à trois sondes distinctes 12, 14 et 16 implantées en divers emplacements du myocarde 18. La sonde 12 est par exemple une sonde endocavitaire auriculaire comportant à son extrémité distale deux actuateurs pour la sélection d'une électrode de stimulation 20 ou 22 correspondante. La sonde 14 est une sonde endocavitaire implantée dans le ventricule, qui intègre à son extrémité distale deux actuateurs permettant la sélection d'une électrode 24 ou 26. Enfin, la sonde 16 est une sonde coronaire de stimulation des cavités gauches, qui intègre à son extrémité distale par exemple quatre actuateurs permettant la sélection de quatre électrodes de stimulation 28, 30, 32 et 34 respectives. Ces diverses sondes peuvent également comporter divers capteurs (non illustrés sur les figures), en particulier pour mesurer l'accélération endocardiaque.

On a représenté de façon plus détaillée sur la Figure 2 l'extrémité de la sonde 16, étant entendu que les deux autres sondes 12 et 14 peuvent être configurées de façon semblable.

Cette sonde 16 inclut deux conducteurs de liaison sous forme de deux conducteurs de liaison en forme de microcâbles 36, 38 qui courent sur toute sa longueur et sont reliés au générateur 10. Ces deux conducteurs 36 et 38 seront désignés par la suite "microcâble proximal" 36 et "microcâble distal" 38, et les bornes correspondantes du générateur seront désignées "distale" et "proximale", par analogie avec le positionnement des deux électrodes d'une sonde bipolaire endocavitaire simple.

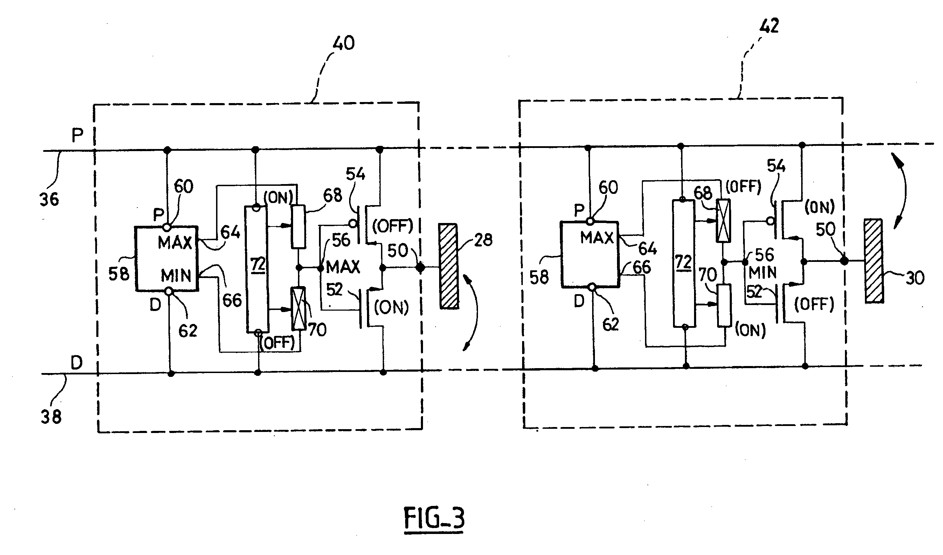

Les microcâbles proximal 36 et distal 38 sont reliés à un ensemble de modules 40, 42, 44, 46, disposés dans la sonde au niveau des électrodes respectives 28, 30, 32, 34. Ces modules 40, 42, 44, 46 peuvent être notamment réalisés sous la forme d'ASICs, dont la structure fonctionnelle interne sera décrite plus en détail en référence à la Figure 3.

Ces modules 40, 42, 44, 46 sont desservis par les microcâbles 36 et 38 à la manière d'un bus, chacun des modules gérant la connexion de l'électrode 28, 30, 32, 34 à laquelle il est associé à l'un ou à l'autre des microcâbles 36 ou 38 (ou à aucun des deux), de manière à définir une configuration de stimulation en fonction du schéma de commutation voulu.

Ainsi, dans le cas d'une stimulation bipolaire, certaines des électrodes 28, 30, 32, 34 seront reliées au microcâble proximal 36, tandis que les autres seront reliées au microcâble distal 38 (ou ne seront éventuellement reliées à aucun des deux microcâbles, si l'électrode n'est pas sélectionnée). Il est possible également de se contenter d'une stimulation monopolaire, en prévoyant de commuter certaines des électrodes à l'un seulement des microcâbles 36 ou 38, l'autre pôle de stimulation étant constitué par le boîtier métallique du générateur 10.

Les divers modules de commutation contrôlée 40, 42, 44, 46 sont identiques et ne diffèrent que par leur adresse de multiplexage, nécessaire pour leur identification au moment de la programmation initiale de la configuration de stimulation.

La Figure 3 illustre plus en détail deux de ces modules, par exemple les modules 40 et 42 associés aux électrodes 28 et 30.

On notera que dans l'exemple illustré chaque module de commutation est associé à une seule électrode, mais on peut également envisager une configuration dans laquelle plusieurs électrodes sont commutées simultanément par un même module, la configuration illustrée n'étant pas limitative.

L'électrode est connectée à une borne d'électrode 50 du module de commutation, qui est également relié aux deux microcâbles proximal 36 et distal 38 par des bornes correspondantes.

L'électrode 28, via la borne 50, peut être connectée alternativement à l'un ou l'autre des deux microcâbles 36 ou 38 par l'intermédiaire de commutateurs commandés 52 et 54, par exemple de type MOS ou MEMS. Dans l'exemple illustré, les interrupteurs commandés 52, 54, sont des MOS complémentaires dont les électrodes de commande sont reliées à un point commun 56. La configuration présente ainsi deux états selon la tension au point 56 :
- pour une tension élevée, l'interrupteur 52 est passant (ON) et l'interrupteur 54 est bloquant (OFF), de sorte que l'électrode 28 se trouve alors mise au potentiel du microcâble distal 38 ;
- lorsque la tension sur le point 56 est faible, la configuration est inverse : l'interrupteur 52 est bloquant (OFF), l'interrupteur 54 est passant (ON), et l'électrode 28 se trouve alors mise au potentiel du microcâble proximal 36.

L'activation des interrupteurs commandés 52, 54, est réalisée grâce à un circuit 58 qui est un générateur de maximum/minimum. Ce circuit 58 est relié en entrée au microcâble proximal 36 par la borne 60 et au microcâble distal 38 par la borne 62. Il délivre en sortie, sur les bornes respectives 64 et 66, la valeur maximum de la tension appliquée entre les microcâbles proximal 36 et distal 38, et sur la borne 66 la tension minimale appliquée entre ces mêmes microcâbles.

Le circuit générateur de maximum/minimum 58 est couplé aux interrupteurs commandés 52 et 54 par l'intermédiaire de composants de mémoire non volatile 68 et 70. Ces composants 68, 70, peuvent prendre deux états selon la manière dont ils ont été programmés, et conservent cet état même en l'absence d'alimentation.

Les composants de mémoire non volatile utilisables dans le cadre de l'invention peuvent être par exemple des cellules à nanotube suspendu telles que celles décrites par exemple dans le US 2009/0310268 A1. Il peut également s'agir de cellules à jonction tunnel magnétique (JTM), qui sont des cellules comprenant une très fine couche d'un matériau isolant ou semiconducteur prise en sandwich entre deux couches ferromagnétiques : si une différence de potentiel est appliquée entre ces deux couches ferromagnétiques, un courant circule à travers la couche isolante, également appelée barrière tunnel ; la valeur de résistance de la jonction, fonction de phénomènes quantiques, peut prendre deux valeurs extrêmes selon la configuration parallèle ou antiparallèle des aimantations des couches ferromagnétiques.

Quelle que soit la technologie choisie pour les composants de mémoire 68, 70, ceux-ci présentent deux états stables, avec un état à faible résistance ou état passant (ON), et un état à résistance élevée ou état bloquant (OFF). L'un ou l'autre état est prédéfini par un circuit de programmation 72 sur la base d'impulsions de programmation spécifiques appliquées sur les microcâbles 36 et 38, par exemple des signaux tels que ceux décrits dans le EP 1 938 861 A1 précité.

La caractéristique essentielle des composants de mémoire 68, 70 est qu'ils sont non volatils, c'est-à-dire que, après qu'ils aient été programmés, ils conservent leur état de façon permanente et sans nécessiter aucune alimentation électrique.

Dans le cas des modules illustrés Figure 3, les deux composants 68, 70 sont programmés de manière que leurs états respectifs soient opposés : l'un est passant lorsque l'autre est bloquant, ou inversement : par exemple pour le module 40 le composant 68 est passant et le composant 70 est bloquant, tandis que pour le module 42 le composant 68 est bloquant et le composant 70 est passant.

Ces composants de mémoire non volatile sont intercalés :
- pour le composant 68, entre la borne de sortie de maximum 64 du générateur de maximum/minimum 58, d'une part, et le point milieu 56 de l'électrode de commande des MOS complémentaires 52, 54, d'autre part ; et
- pour le composant 70 : entre la borne de sortie de minimum 66 du générateur de maximum/minimum 58, d'une part, et le point milieu 56 de l'électrode de commande des MOS complémentaires 52, 54, d'autre part.

Dans l'exemple illustré sur la Figure 3, pour le module de commutation 40 où la mémoire non volatile 68 est passante, c'est la tension de maximum, présente sur la borne 64, qui sera appliquée au point milieu 56 de commande des MOS 52, 54, ce qui aura pour effet de rendre passant le MOS 52 et bloquant le MOS (complémentaire) 54.

Pour le module de commutation 42, la situation est inverse : le fait que le composant de mémoire non volatile 70 soit passant fait en sorte que c'est la tension de minimum présente sur la borne 66 qui est appliquée aux électrodes de commande des MOS 52, 54, rendant passant le MOS (inversé) 54 et bloquant le MOS 52.

Ainsi, l'électrode 30 sera mise au potentiel du microcâble proximal 36, tandis que l'électrode 28 sera mise au potentiel de l'électrode distale 38.

On peut obtenir avec cette configuration une stimulation bipolaire entre les deux électrodes 28 et 30 (et de même avec les autres électrodes éventuellement présentes de la sonde), grâce à la combinaison, selon l'invention, (i) d'interrupteurs commandés volatils (les MOS 52, 54) avec (ii) des composants de mémoire non volatile (les cellules de mémoire 68, 70). L'absence d'alimentation de la sonde, par exemple lors d'un remplacement de celle-ci, n'entraînera aucune déprogrammation de la configuration de commutation, qui n'aura pas à être reconstituée par exemple après échange ou déconnexion du générateur.

Pour une sonde multiélectrode, il est possible de connecter n'importe laquelle des électrodes soit à la borne proximale soit à la borne distale du générateur, ce qui procure ainsi un très grand nombre de configurations possibles et donc une plus grande flexibilité pour une meilleure thérapie du patient. Surtout, une fois qu'une configuration a été fixée et programmée, il ne sera plus nécessaire de reconfigurer périodiquement les modules, ni de maintenir leurs alimentations.

On notera que, de façon avantageuse, le courant qui passe dans les cellules de mémoire non volatile 68, 70 est un courant faible, ce qui permet d'utiliser des technologies telles que celles évoquées plus haut de cellules à nanotube suspendu ou à jonction tunnel magnétique, qui ne supportent qu'un courant très faible. Le courant de stimulation proprement dit ne passe pas par ces cellules de mémoire, mais par les interrupteurs commandés MOS 52, 54, dont la résistance Rₒₙ à l'état fermé est faible. Le courant susceptible d'être délivré n'est donc pas limité par la technologie choisie, et d'autre part, du fait de la faible résistance Rₒₙ à l'état passant, l'impédance vue depuis le générateur n'est pas sensiblement modifiée.

Enfin, on notera que la stimulation ne peut en aucun cas déprogrammer ni détruire les cellules de mémoire non volatile 68, 70, de sorte qu'il n'est nécessaire de prendre aucune mesure de protection particulière en ce qui les concerne.

## Revendications

1. Un module de commutation contrôlée d'électrode, pour un dispositif médical implantable actif comprenant un générateur (10) et une sonde (12, 14, 16) reliée à ce générateur et pourvue d'une pluralité d'électrodes (28, 30, 32, 34) sélectivement commutables de détection/stimulation, ce module (40, 42, 44, 46) comprenant :
- au moins une borne proximale et une borne distale, aptes à être couplées à des conducteurs respectifs (36, 38) d'une ligne bifilaire de la sonde, reliée au générateur ;
- une borne d'électrode (50), reliée à l'une desdites électrodes (28, 30, 32, 34) de détection/stimulation de la sonde ;
- un circuit de commutation sélective de la borne d'électrode à l'une ou à l'autre des bornes proximale et distale, comprenant au moins un interrupteur commandé couplant la borne d'électrode (50) respectivement aux bornes proximale et distale, et associé à au moins un composant de mémoire non volatile programmable ; et
- un circuit (72) de décodage de signaux délivrés par le générateur et transmis par la ligne bifilaire pour commander le circuit de commutation selon une configuration particulière correspondante,
module **caractérisé en ce que** :
- il est prévu deux interrupteurs commandés (52,54) couplant la borne d'électrode (50) respectivement aux bornes proximale et distale, ces interrupteurs commandés étant des interrupteurs volatils nécessitant pour leur activation une alimentation électrique ;
- ledit composant de mémoire non volatile programmable (68, 70) possède en l'absence d'alimentation électrique deux états stables, chacun des ces états stables contrôlant un état correspondant ouvert ou fermé de l'interrupteur commandé ;
- le circuit de décodage (72) comporte des moyens pour programmer préalablement l'état du composant de mémoire non volatile (68, 70) par des signaux correspondants appliqués par le générateur sur la ligne bifilaire ; et
- le circuit de commutation comprend en outre un circuit générateur de maximum-minimum (58), couplé en entrée entre les deux bornes proximale et distale, et couplé en sortie aux interrupteurs commandés (52, 54) pour commander sélectivement ceux-ci via le composant de mémoire non volatile (68, 70) correspondant.

2. Le module de commutation de la revendication 1, dans lequel les deux interrupteurs commandés (52, 54) sont des interrupteurs complémentaires commandés par application d'une tension à une borne de pilotage commune, de sorte que lorsque l'un des interrupteurs est commandé ouvert l'autre soit commandé fermé, et *vice versa.*

3. Le module de commutation de la revendication 2, comprenant deux composants de mémoire non volatile (68, 70) montés en série entre une sortie de minimum (66) et une sortie de maximum (64) du générateur de maximum-minimum (58), avec l'un des composants de mémoire non volatile programmé dans l'un de ses états stables et l'autre dans l'état stable opposé, et le point milieu (56) du montage série étant relié à la borne de pilotage commune des interrupteurs complémentaires.

4. Le module de commutation de la revendication 1, dans lequel le composant de mémoire non volatile programmable (68, 70) est une cellule à nanotube suspendu.

5. Le module de commutation de la revendication 1, dans lequel le composant de mémoire non volatile programmable (68, 70) est une cellule à jonction tunnel magnétique.

6. Une sonde pour un dispositif médical implantable actif comprenant un générateur (10) et ladite sonde (12, 14, 16) reliée à ce générateur, cette sonde comprenant :
- un corps de sonde allongé, comprenant une ligne bifilaire (36, 38) avec des conducteurs respectifs reliés au générateur ;
- une pluralité d'électrodes (28, 30, 32, 34) de détection/stimulation sélectivement commutables ; et
- un module de commutation contrôlée (40, 42, 44, 46) apte à coupler sélectivement chacune des électrodes à l'une ou à l'autre des conducteurs de la ligne bifilaire,
sonde **caractérisée en ce que** ledit module de commutation contrôlée (40, 42, 44, 46) est un module selon l'une des revendications 1 à 5.

## Patentansprüche

1. Modul zum gesteuerten Kommutieren von Elektroden für eine aktive implantierbare medizinische Vorrichtung, die einen Generator (10) und eine mit diesem Generator verbundene Sonde (12, 14, 16) umfasst und mit mehreren Elektroden (28, 30, 32, 34) versehen ist, die wahlweise zwischen Detektion und Stimulation kommutierbar sind, wobei dieses Modul (40, 42, 44, 46) Folgendes umfasst:
- wenigstens einen proximalen Anschluss und einen distalen Anschluss, die mit entsprechenden Leitern (36, 38) einer Doppeldrahtleitung der Sonde, die mit dem Generator verbunden ist, gekoppelt werden können;
- einen Elektrodenanschluss (50), der mit einer der Detektions-/Stimulationselektroden (28, 30, 32, 34) der Sonde verbunden ist;
- eine Schaltung zum wahlweisen Kommutieren des Elektrodenanschlusses zu dem einen oder dem anderen des proximalen und des distalen Anschlusses, die wenigstens einen gesteuerten Ein/Aus-Schalter umfasst, der den Elektrodenanschluss (50) mit dem proximalen bzw. dem distalen Anschluss koppelt und wenigstens einer programmierbaren, nichtflüchtigen Speicherkomponente zugeordnet ist; und
- eine Schaltung (72) zum Decodieren von Signalen, die von dem Generator ausgegeben werden und über die Doppeldrahtleitung übertragen werden, um die Kommutationsschaltung gemäß einer entsprechenden bestimmten Konfiguration zu steuern,
wobei das Modul **dadurch gekennzeichnet ist, dass**:
- zwei gesteuerte Ein/Aus-Schalter (52, 54) vorgesehen sind, die den Elektrodenanschluss (50) mit dem proximalen bzw. dem distalen Anschluss koppeln, wobei diese gesteuerten Ein/Aus-Schalter flüchtige Ein/Aus-Schalter sind, die für ihre Aktivierung eine elektrische Versorgung benötigen;
- die programmierbare nichtflüchtige Speicherkomponente (68, 70) bei Abwesenheit einer elektrischen Versorgung zwei stabile Zustände besitzt, wobei jeder dieser stabilen Zustände einen entsprechenden offenen oder geschlossenen Zustand des gesteuerten Ein/Aus-Schalters steuern;
- die Decodierungsschaltung (72) Mittel umfasst, um im Voraus den Zustand der nichtflüchtigen Speicherkomponente (68, 70) durch entsprechende Signale zu programmieren, die durch den Generator an die Doppeldrahtleitung angelegt werden; und
- die Kommutationsschaltung außerdem eine Maximum/Minimum-Generatorschaltung (58) umfasst, die mit dem Eingang zwischen den proximalen und den distalen Anschluss geschaltet ist und mit dem Ausgang mit dem gesteuerten Ein/Aus-Schaltern (52, 54) verbunden ist, um diese wahlweise über die entsprechende nichtflüchtige Speicherkomponente (68, 70) zu steuern.

2. Kommutationsmodul nach Anspruch 1, wobei die zwei gesteuerten Ein/Aus-Schalter (52, 54) komplementäre Ein/Aus-Schalter sind, die durch Anlegen einer Spannung an einen gemeinsamen Steueranschluss gesteuert werden, derart, dass dann, wenn einer der Ein/Aus-Schalter nach offen gesteuert ist, der andere nach geschlossen gesteuert wird und umgekehrt.

3. Kommutationsmodul nach Anspruch 2, das zwei nichtflüchtige Speicherkomponenten (68, 70) umfasst, die zwischen einem Minimumausgang (66) und einem Maximumausgang (64) des Maximum/Minimum-Generators (58) in Reihe geschaltet sind, wobei eine der programmierten nichtflüchtigen Speicherkomponenten in einem ihrer stabilen Zustände ist und die Andere im entgegengesetzten stabilen Zustand ist, wobei der Mittelpunkt (56) der Reihenschaltung mit dem gemeinsamen Steueranschluss der komplementären Ein/Aus-Schalter verbunden ist.

4. Kommutationsmodul nach Anspruch 1, wobei die programmierbare nichtflüchtige Speicherkomponente (68, 70) eine Zelle mit aufgehängter Nanoröhre ist.

5. Kommutationsmodul nach Anspruch 1, wobei die programmierbare nichtflüchtige Speicherkomponente (68, 70) eine Zelle mit magnetischem Tunnelübergang ist.

6. Sonde für eine aktive implantierbare medizinische Vorrichtung, die einen Generator (10) und die mit diesem Generator verbundene Sonde (12, 14, 16) umfasst, wobei diese Sonde umfasst:
- einen langgestreckten Sondenkörper, der eine Doppeldrahtleitung (36, 38) enthält, deren jeweilige Leiter mit dem Generator verbunden sind;
- mehrere Detektions-/Stimulationselektroden (28, 30, 32, 34), die wahlweise kommutierbar sind; und
- ein Modul für gesteuerte Kommutation (40, 42, 44, 46), das jede der Elektroden mit dem einen oder dem anderen der Leiter der Doppeldrahtleitung wahlweise verbinden kann,
wobei die Sonde **dadurch gekennzeichnet ist, dass** das Modul (40, 42, 44, 46) für gesteuerte Kommutation ein Modul nach einem der Ansprüche 1 bis 5 ist.

## Claims

1. A lead controlled-switching module for an active implantable medical device comprising a generator (10) and a lead (12, 14, 16) connected to this generator and provided with a plurality of selectively switchable detection/stimulation electrodes (28, 30, 32, 34), said module (40, 42, 44, 46) comprising:
- at least one proximal terminal and one distal terminal, operable to be coupled to respective conductors (36, 38) of a two-wire line of the lead, connected to the generator;
- one electrode terminal (50), connected to one of said detection/stimulation electrodes (28, 30, 32, 34) of the lead;
- a circuit for selectively switching the electrode terminal to one or the other of the proximal and distal terminals, comprising at least one controlled switch coupling the electrode terminal (50) to the proximal and distal terminals, respectively, and associated to at least one non-volatile programmable memory component; and
- a circuit (72) for decoding the signal delivered by the generator and transmitted through the two-wire line, to control the switching circuit according to a corresponding particular configuration,
said module being **characterized in that**:
- two controlled switches (52, 54) are provided, coupling the electrode terminal (50) to the proximal and distal terminals, respectively, such controlled switches being volatile switches requiring an electric power supply for their activation;
- said programmable non-volatile memory component (68, 70) has, in the absence of electric power supply, two stable states, each of these stable states controlling a corresponding open or closed state of the controlled switch;
- the decoding circuit (72) includes means for previously programming the state of the non-volatile memory component (68, 70) by corresponding signals applied by the generator to the two-wire line; and
- the switching circuit further comprises a maximum-minimum generator circuit (58), coupled at the input between the two proximal and distal terminals, and coupled at the output to the controlled switches (52, 54) to selectively control them via the corresponding non-volatile memory component (68, 70).

2. The switching module of claim 1, wherein the two controlled switches (52, 54) are complementary switches controlled by application of a voltage to the common piloting terminal, so that, when one of the switches is controlled open, the other is closed, and *vice versa.*

3. The switching module of claim 2, comprising two non-volatile memory components (68, 70) mounted in series between a minimum output (66) and a maximum output (64) of the maximum-minimum generator (58), with one of the non-volatile memory component programmed into one of said stable states and the other in the opposite stable state, and the central point (56) of the series mounting being connected to the common piloting terminal of the complementary switches.

4. The switching module of claim 1, wherein the programmable non-volatile memory component (68, 70) is a suspended nanotube cell.

5. The switching module of claim 1, wherein the programmable non-volatile memory component (68, 70) is a magnetic tunnel junction cell.

6. A lead for an active implantable medical device comprising a generator (10) and said lead (12, 14, 16) connected to this generator, said lead comprising :
- an elongated lead body, comprising a two-wire line (36, 38) with respective conductors connected to the generator;
- a plurality of selectively switchable detection / stimulation electrodes (28, 30, 32, 34); and
- a controlled switching module (40, 42, 44, 46) adapted to couple selectively each of the electrodes to one or the other of the conductors of the two-wire line,
said probe being **characterized in that** said controlled switching module (40, 42, 44, 46) is a module according to one of claims 1 to 5.
